# EUROPEAN PATENT APPLICATION

(11) **EP 4 057 289 A1**
(43) Date of publication of application: **14.09.2022**
(21) Application number: 19951705.3
(22) Date of filing: 07.11.2019
(51) Int. Cl.: G16H 20/00

(54) **METHOD FOR PROVIDING LIFESTYLE IMPROVEMENT**

(71) Applicant: A10 Lab. Inc., Shibuya-ku Tokyo 150-0013 (JP)
(72) Inventor: NAGASAKA, Go, Tokyo 150-0013 (JP)
(74) Representative: Herrero & Asociados, S.L.
(86) International application number: PCT/JP2019/043756
(87) International publication number: WO 2021/090452

(57) **Abstract**

An object is to realize a lifestyle habit improvement method capable of further improving usability. In a providing method of lifestyle habit improvement according to an aspect of the present invention, improvement is provided for a user belonging to a team in which a plurality of users communicate via an interface for chat communication, the method comprising, by a server terminal: accepting, from a user terminal, a request for selecting a desired team by a user; enrolling the user in the team; accepting, from the user terminal, lifestyle habit information of the user for every predetermined period; and storing the lifestyle habit information in a user data storage of the server terminal.

## Description

### Technical Field

The present invention relates to a providing method of lifestyle habit improvement for a user.

### Background Art

In recent years, services for managing and/or improving lifestyle habits through communication of a user with an instructor via an application installed in a user terminal such as a smartphone or a tablet of the user have been prevailing.

For example, Patent Literature 1 discloses a sleep management system which performs sessions based on cognitive behavior therapy including a process of questions and answers on sleep between an information processing server and an information processing terminal device.

### Citation List

### Patent Literature

Patent Literature 1: Japanese Patent Laid-Open No. 2018-186362

### Summary of Invention

### Technical Problem

There is however practical difficulty, in Patent Literature 1, in a user's recalling and answering their own sleep situations upon reception of a set of questions while usability is improved therein in capability of acquiring information regarding the sleep of the user with the questions and answers repeated via a chat communication interface, and the same problem remains with respect to other lifestyle habits.

Therefore, an object of the present invention is to realize a lifestyle habit improvement method capable of further improving usability.

### Solution to Problem

In a providing method of lifestyle habit improvement according to an aspect of the present invention, improvement is provided for a user belonging to a team in which a plurality of users communicate via an interface for chat communication, the method comprising, by a server terminal: accepting, from a user terminal, a request for selecting a desired team by a user; enrolling the user in the team; accepting, from the user terminal, lifestyle habit information of the user for every predetermined period; and storing the lifestyle habit information in a user data storage of the server terminal.

### Advantageous Effects of Invention

According to the present invention, there can be realized a lifestyle habit improvement method capable of further improving usability.

### Brief Description of Drawings

[Figure 1] Figure 1 is a block configuration diagram showing a system according to a first embodiment of the present invention, the system providing a lifestyle habit improvement method.
[Figure 2] Figure 2 is a functional block configuration diagram showing a server terminal 100 in Figure 1.
[Figure 3] Figure 3 is a functional block configuration diagram showing a user terminal 200 in Figure 1.
[Figure 4] Figure 4 is a diagram showing an example of user data stored in the server 100.
[Figure 5] Figure 5 is a diagram showing an example of team data stored in the server 100.
[Figure 6] Figure 6 is an example of a flowchart showing a providing method of lifestyle habit improvement according to the first embodiment of the present invention.
[Figure 7] Figure 7 shows diagrams showing an example of a user interface screen according to the first embodiment of the present invention, the screen relating to team selection.
[Figure 8] Figure 8 shows diagrams showing an example of a user interface screen according to the first embodiment of the present invention, the screen showing acceptance of lifestyle habit information.
[Figure 9] Figure 9 is an example of a flowchart showing a displaying method of a lifestyle habit log in the providing method of lifestyle habit improvement according to the first embodiment of the present invention.
[Figure 10] Figure 10 shows diagrams showing an example of a user interface screen according to the first embodiment of the present invention, the screen showing a lifestyle habit log.
[Figure 11] Figure 11 is a diagram showing an example of a user interface screen according to the first embodiment of the present invention, the screen showing the number of steps.
[Figure 12] Figure 12 shows diagrams showing an example of a user interface screen according to the first embodiment of the present invention, the screen showing meals.
[Figure 13] Figure 13 shows diagrams showing an example of a user interface screen according to the first embodiment of the present invention, the screen showing a body weight.

### Description of Embodiments

Hereafter, embodiments of the present invention will be described with reference to the drawings. Note that the embodiments described below do not unreasonably limit the matters of the present invention disclosed in the claims. Moreover, the components shown for any embodiment are not entirely the essential components for the present invention.

### <Configuration>

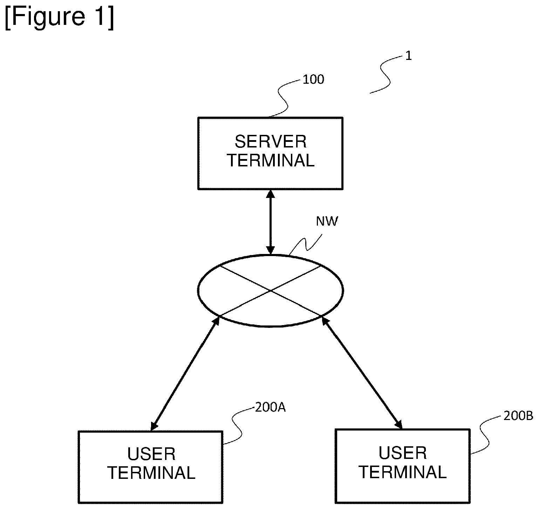

Figure 1 is a block configuration diagram showing a system according to a first embodiment of the present invention, the system providing lifestyle habit improvement. This system 1 has a server terminal 100 and a plurality of user terminals 200A and 200B, they providing a service to prompt a plurality of users to improve lifestyle habits by the users making communication via an interface for chat communication, the user terminals being associated with the respective users as customers of the service.

The server terminal 100 and the user terminals 200A and 200B are connected to one another via a network NW. The network NW is composed of the Internet, an intranet, a wireless LAN (Local Area Network), a WAN (Wide Area Network), and/or the like.

The server terminal 100 generates, by users as customers of the service or a service provider, a plurality of chat groups each called a "team" composed of a plurality of users. Each team is associated with predetermined categories associated with a habit the goal of which the users want to achieve, and is composed of user members the upper limit number of which is defined (for example, five). Each of the users aims to achieve the goal (for example, "to lose 10 kg of body weight") shared in the team while they are posting messages and images regarding the challenges to achieve the goal and mutually encouraging the other users via a chat communication interface for the team. The server terminal 100 may be a general-purpose computer such, for example, as a workstation or a personal computer, or may be logically implemented through cloud computing. While in the present embodiment, one server terminal is exemplarily presented for convenience of description, there may be a plurality of those with no limitation.

While each user terminal 200 is an information processing device such, for example, as a personal computer or a tablet terminal which is associated with (for example, owned by) a customer user using a service to provide lifestyle habit improvement provided by the server terminal 100, it may be composed of a smartphone, a mobile phone, a PDA, or the like.

While in the present embodiment, the system 1 is described as a configuration, including the server terminal 100 and the user terminals 200A and 200B, with which the users use the respective terminals to make operations for the server terminal 100, the server terminal 100 may be configured in a stand-alone manner, so that the server terminal itself has functions directly operated by the users.

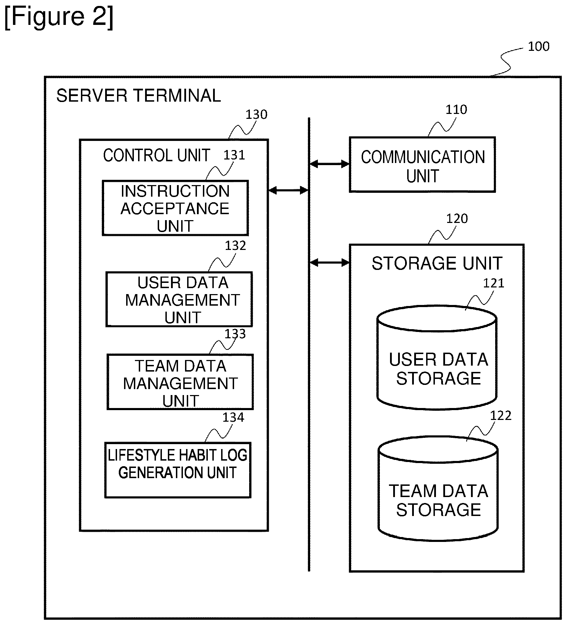

Figure 2 is a functional block configuration diagram of the server terminal 100 in Figure 1. The server terminal 100 includes a communication unit 110, a storage unit 120, and a control unit 130.

The communication unit 110 is a communication interface for communicating with the user terminals 200, a picture contributor terminal 300 and a sightseeing spot user terminal 400 via the network NW, and the communication is performed under a communication protocol such, for example, as the TCP/IP (Transmission Control Protocol/Internet Protocol).

The storage unit 120 stores programs for executing various kinds of control processing and functions in the control unit 130, input data, and the like, and is composed of a RAM (Random Access Memory), a ROM (Read Only Memory), and the like. Moreover, the storage unit 120 has a user data storage 121 which stores various kinds of data regarding the users, and a team data storage 122 which stores various kinds of data regarding the team. Note that a database (not shown) which stores such various kinds of data may be constructed outside the storage unit 120 or the server terminal 100.

The control unit 130 controls the entire operation of the server terminal 100 by executing the program(s) stored in the storage unit 120 and is composed of a CPU (Central Processing Unit), a GPU (Graphics Processing Unit), and the like. The control unit 130 has, as its functions, an instruction acceptance unit 131 which accepts instructions from the user terminals 200 and the like, a user data management unit 132 which refers to and processes the various kinds of data regarding the users, a team data management unit 133 which refers to and processes the various kinds of data regarding the team, and a lifestyle habit log generation unit 134 which generates lifestyle habit logs on the basis of lifestyle habit information accepted from the users. These instruction acceptance unit 131, user data management unit 132, team data management unit 133, and lifestyle habit log generation unit 134 are initiated by the program(s) stored in the storage unit 120 and performed by the server terminal 100 as a computer (electronic calculator).

The instruction acceptance unit 131 accepts an instruction from each user terminal 200 via the communication unit 110 when the user makes a predetermined request (by inputting text, pressing an icon, or making the similar action) via a user interface such as a screen which is provided by the server terminal 100 and displayed on the user terminal 200 through a web browser or an application.

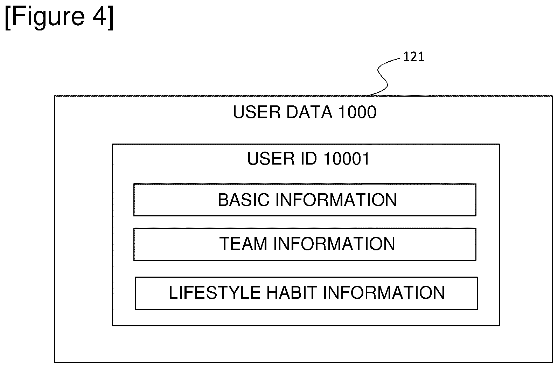

The user data management unit 132 manages the various kinds of data regarding the users (such, for example, as user IDs, basic information of the users, information of the team which the users belong to, and lifestyle habit information transmitted by the users shown in Figure 4) and performs processing on those.

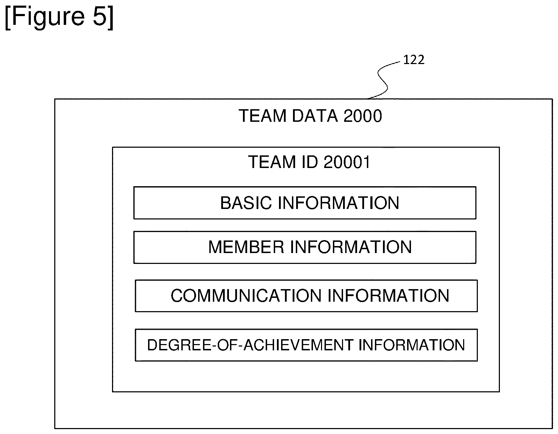

The team data management unit 133 manages the various kinds of data regarding the team (such, for example, as a team ID, basic information of the team, communication information regarding the contents of the messages posted through the chat communication interface for the team, and information regarding the degree of achievement of the goal shared in the team shown in Figure 5) and performs processing on those.

The lifestyle habit log generation unit 134 generates, in response to requests from the users or regardless of such requests, lifestyle habit logs on the basis of the lifestyle habit information stored in the user data storage 121 and transmitted by the users, and performs processing to display them on the user terminals 200.

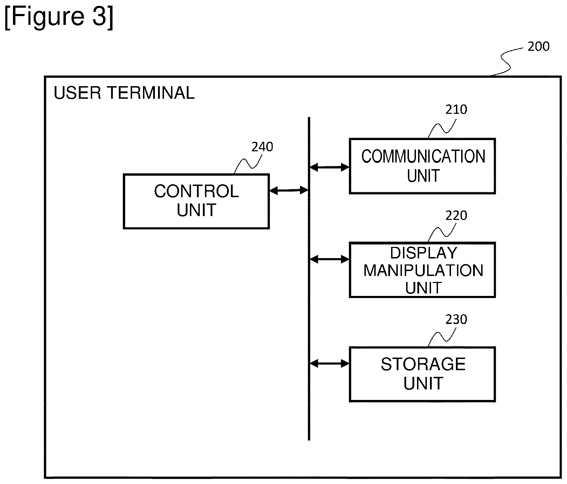

Figure 3 is a functional block configuration diagram showing the user terminal 200 in Figure 1. The user terminal 200 includes a communication unit 210, a display manipulation unit 220, a storage unit 230, and a control unit 240.

The communication unit 210 is a communication interface for communicating with the server terminal 100 via the network NW, and the communication is performed under a communication protocol such, for example, as the TCP/IP.

The display manipulation unit 220 is a user interface used for the user inputting an instruction and for displaying text, images, and the like in response to input data from the control unit 240, when the user terminal 200 is composed of a personal computer, is composed of a display, and a keyboard and/or a mouse, and when the user terminal 200 is composed of a smartphone or a tablet terminal, is composed of a touch panel and/or the like. This display manipulation unit 220 is initiated by a control program stored in the storage unit 230 and performed by the user terminal 200 as a computer (electronic calculator).

The storage unit 230 stores programs for executing various kinds of control processing and functions in the control unit 240, input data, and the like, and is composed of a RAM, a ROM, and the like. Moreover, the storage unit 230 temporarily stores the contents of communication with the server terminal 100.

The control unit 240 controls the entire operation of the user terminal 200 by executing the program(s) stored in the storage unit 230 and is composed of a CPU, a GPU, and the like.

Note that there may be employed a configuration including the function of the display manipulation unit in the server terminal 100, an in this case, there may be employed the configuration that does not include the user terminals 200.

Figure 4 is a diagram showing an example of user data stored in the server 100.

User data 1000 shown in Figure 4 stores the various kinds of data regarding the users. While Figure 4 shows an example of one user (user identified by a user ID "10001") for convenience of description, information of a plurality of users can be stored. The various kinds of data regarding each user can include the basic information of the user (a user password, the name, the age, the sexuality, SNS information, and the membership status (a fee-free membership user or a premium membership user) of the user, and status information (for example, a "badge") given based on posts of text and images associated with missions in the team that it belongs to), the information of the team that it belongs to (a team ID, a team name, and the like), and the lifestyle habit information (the number of steps (steps), a body weight (kg), meals (kcal), consumed calories (kcal), a sleeping time (hours; minutes), image data, text data, various kinds of data associated with dates in a calendar, and the like), for example.

Figure 5 is a diagram showing an example of team data stored in the server 100.

Team data 2000 shown in Figure 5 stores the various kinds of data regarding the team. While Figure 5 shows an example of one team (team identified by a team ID "20001") for convenience of description, information of a plurality of teams can be stored. The various kinds of data regarding each team can include the basic information of the team (a team name, age restriction, sexuality restriction, an active period, an automatic leaving period, an assistant character, tag information, and the like), information of the members belonging to it (user IDs, user names, and the like), communication information (histories of messages and images posted to the chat communication interface for the team, and the like), and degree-of-achievement information (information shared in the team and regarding the degrees of achievement of the daily goals and the final goal, and the like), for example.

### <Flow of Processing>

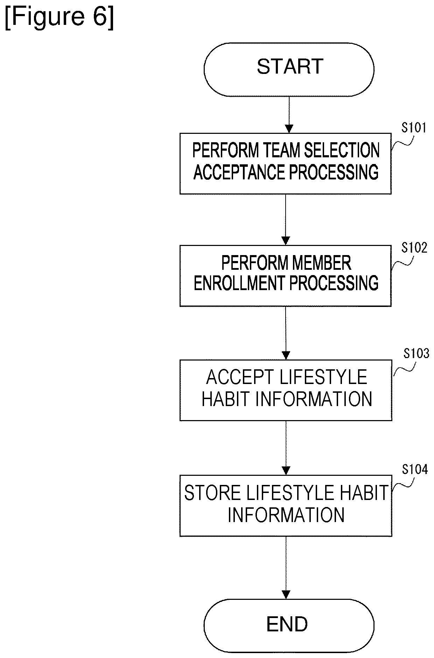

Referring to Figure 6, there is described a flow of processing of a providing method of lifestyle habit improvement performed by the system 1 of the present embodiment. Figure 6 is an example of a flowchart according to the providing method of lifestyle habit improvement according to the first embodiment of the present invention.

Here, in order to use this system 1, a user accesses the server terminal 100 using the web browser, the application or the like of the user terminal 200, and when it uses the service for the first time, it inputs the basic information of the user and the like mentioned above, or when it has already taken a user account, it makes a login through predetermined authentication such, for example, as input of its ID and password, which thereby makes the service available. After this authentication, a predetermined user interface is provided through a web site, an application or the like, the process being put forward to step S101 shown in Figure 6.

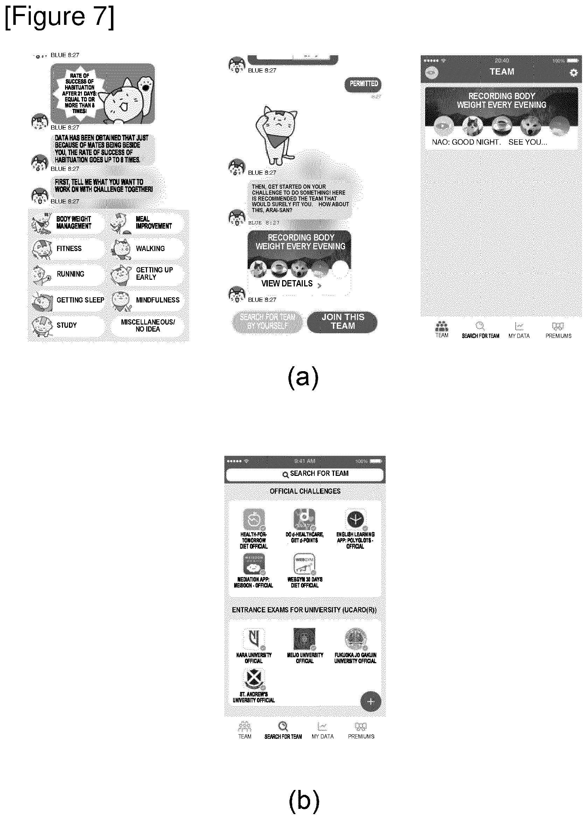

First, as the processing of step S101, the instruction acceptance unit 131 of the server terminal 100 receives a request for selecting a desired team from the user terminal 200 via the communication unit 110. For example, as shown in Figure 7, the server terminal 100 presents some teams to be selected for the user in some manners on user interface screens each displayed on the user terminal 100. For example, as shown in Figure 7(a), it can recommend a team on the basis of the contents of the user's answers with respect to some questions for the user on a tutorial screen that is for a beginner user. For example, it can recommend a team the goal of which is to "record the body weight every evening" when it questions the user about something to want to work on and the user answers "body weight management". Otherwise, the server terminal 100 can present team candidates satisfying conditions through a keyword search request. When the user selects the desired team, the selection request for the team is transmitted to the server terminal 100 from the user terminal 200 via the network.

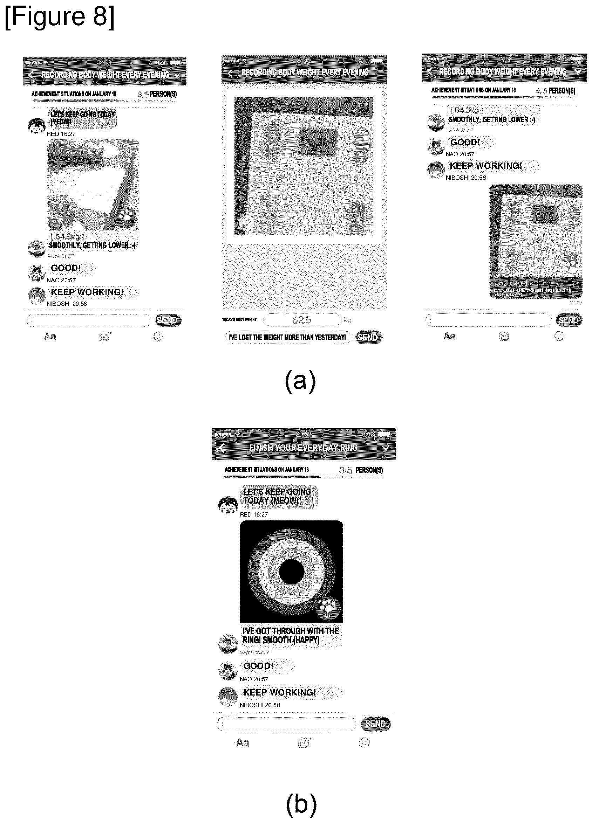

Here, each team is associated with major categories such as "Recommended", "Diet", and "Fitness", and, for example, for the major category "Fitness", minor categories such as "Muscular Workout", "Walking", and "Walking Relay". Moreover, a team can be generated by an individual user as a customer of the service, or a corporate user such as a service enterprise or a partner, and the corporate user, in particular, that is a partner can generate major categories relevant to the generated team (such, for example, as a "University Entrance Examination" category shown in Figure 8(b)) or minor categories (such, for example, as an "ABC App Official" category generated in the "Diet" category) in order to advertise/promote the sale of merchandises and services provided by that corporate. Thereby, the users can take a shortcut to access the team generated by the corporate user.

Next, as the process of step S102, the user data processing unit 132 of the server terminal 100 refers to the user information of transmission of the selection request from the user data 1000 stored in the user data storage 121, and the team data processing unit 133 refers to the team data 2000 stored in the team data storage 122 on the basis of the user information referred to and performs processing of enrolling the user in the relevant team. For example, when the server terminal 100 accepts a request for selecting a team named "recording the body weight every evening" from a user X, it performs processing of enrolling the user X in "recording the body weight every evening".

Next, as the processing of step S103, the instruction acceptance unit 131 of the server terminal 100 accepts the lifestyle habit information from the user terminal 200 via the communication unit 110. The server terminal 100 accepts text and images regarding the lifestyle habits posted via the chat communication interface for the team which is displayed on the user terminal 100, for every predetermined period. For example, as shown in Figure 8(a), in the chat communication interface, when the user posts an image of a body weight meter, a region which the body weight value (kg) is entered in and a region which a message is entered in are displayed. The body weight value (kg) can also be extracted from the captured image as text data through OCR processing or the like and be automatically input. Moreover, input items (such, for example, as "today's body weight") can also be displayed according to the goal shared in the team (for example, "recording the body weight every evening"). When accepting the lifestyle habit information from the user, the server terminal 100 displays the accepted information (for example, the body weight value (kg) input along with the image of the body weight meter (together with the message)), in the chat communication interface. Each user of the team views images and text posted by another user to confirm that this user has cleared a predetermined mission (for example, to measure the body weight every evening) with respect to the goal, and takes some action (for example, to make a stamp of a cat footprint) in order to prove the confirmation. According to these actions, the degree of achievement for the goal set by the team (or the goal of the day to be cleared for the goal) can be updated and visually displayed. Moreover, as shown in Figure 8(b), one user which the team is composed of can also visually display the degree of achievement for itself as an individual using a predetermined image (acquired from the server terminal 100 or an external resource).

Next, as the processing of step S104, the user data management unit 132 of the server terminal 100 stores the accepted lifestyle habit information, associating it with the relevant user, in the user data 1000 stored in the user data storage 121. Herein, the user data management unit 132 can store the lifestyle habit information (for example, the body weight value) acquired from the user, associating it with the date. Moreover, by storing image data of meals, associating them with the date and predetermined hours, they can be sorted out into the morning, the noon, and the evening.

As above, in a user group in which a plurality of users have the common goal and the chat communication is enabled among them, the user can take continuous actions for achieving the goal by posting appointed lifestyle habit information for every predetermined period under mutual communication for achieving the goal, and can continuously acquire the information regarding the lifestyle habits and acquire the history thereof comfortably under such communication.

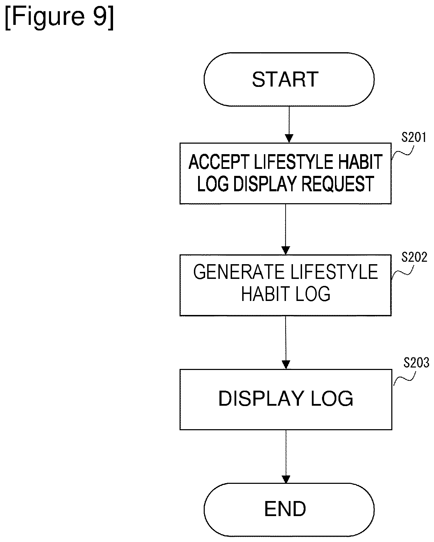

Figure 9 is an example of a flowchart showing a displaying method of the lifestyle habit log in the providing method of lifestyle habit improvement according to the first embodiment of the present invention.

First, in S201, the instruction acceptance unit 131 of the server terminal 100 accepts a request for displaying the lifestyle habit log from the user terminal 200 via the communication unit 110. For example, the user inputs date information or selects a desired data within a calendar display in a predetermined user interface displayed on the user terminal 100.

Next, in S202, the lifestyle habit log generation unit 134 generates the lifestyle habit log corresponding to the selected date. For example, the user data management unit 132 refers to the lifestyle habit information of the relevant user corresponding to the selected date in the user data stored in the user data storage 121, and the lifestyle habit log generation unit 134 generates the lifestyle habit log on the basis of the information referred to by the user data management unit 132.

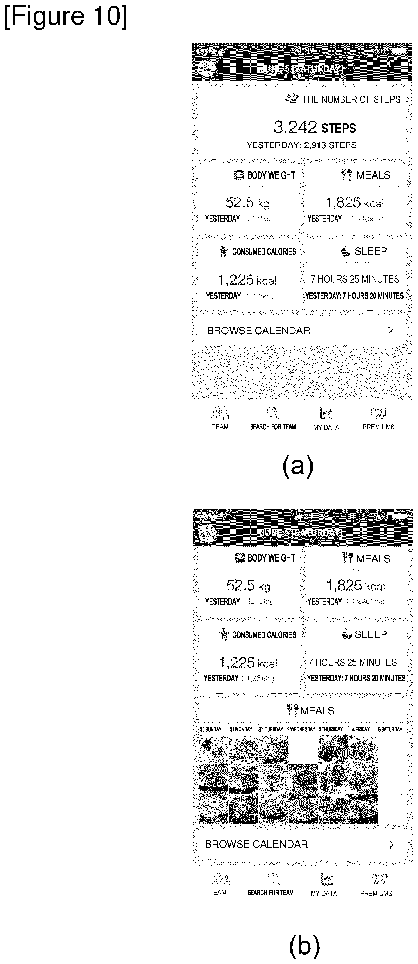

Next, in S203, the lifestyle habit log generation unit 134 causes the user terminal 100 to display the generated lifestyle habit log. For example, as shown in Figure 10(a), when the user selects the date "June 5 (Saturday)", the lifestyle habit log generation unit 134 can generate the lifestyle habit information as the lifestyle habit log on the basis of the lifestyle habit information corresponding to this date, and configure information required for generate a screen to be displayed via the user interface of the user terminal 100. For example, as shown in Figure 10(a), via the user interface of the user terminal 100, there are displayed, as the lifestyle habit log, the lifestyle habit information regarding the "number of steps", the "body weight", the "consumed calories", the "meals", and the "sleep" of the user on "June 5 (Saturday)", and as shown in Figure 10(b), there can also be displayed, as the lifestyle habit information, images obtained by photographing the meals on the basis of the corresponding date and hours in a calendar display region.

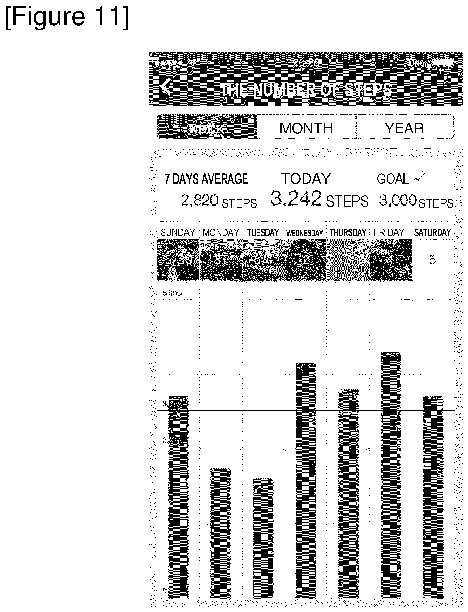

Moreover, for example, when the user selects the "number of steps" in the display of the lifestyle habit log shown in Figure 10, the transition of the numbers of steps for seven days among which "June 5 (Saturday)" is the final day can also be visualized and displayed, and moreover, the average value of those can also be displayed, as shown in Figure 11. Furthermore, as shown in Figure 11, the transition and the average value of the numbers of steps can also be displayed for the period of a "month" or a "year" in addition to a "week".

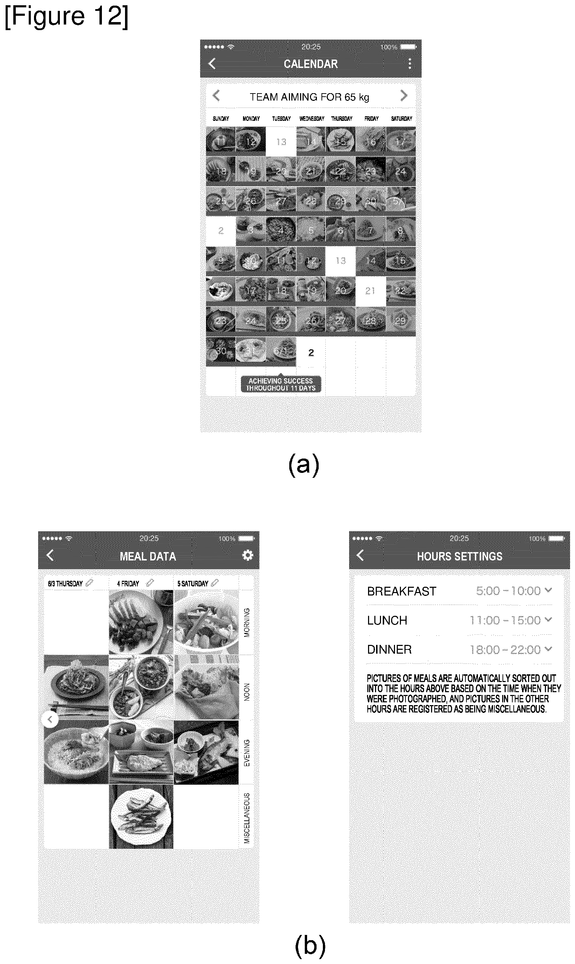

Moreover, for example, when the user selects the "calendar" in the display of the lifestyle habit log shown in Figure 10, the images posted by the user can be displayed as a list so as to correspond to the dates in the calendar as shown in Figure 12(a). Moreover, the images of the meals can be displayed such that they correspond to breakfasts, lunches, dinners, and other hours (snacks) and correspond to the dates in the calendar as shown in Figure 12(b). Here, the user can freely configure the hours of breakfast, lunch, and dinner such that they meet the lifestyle and can also change them.

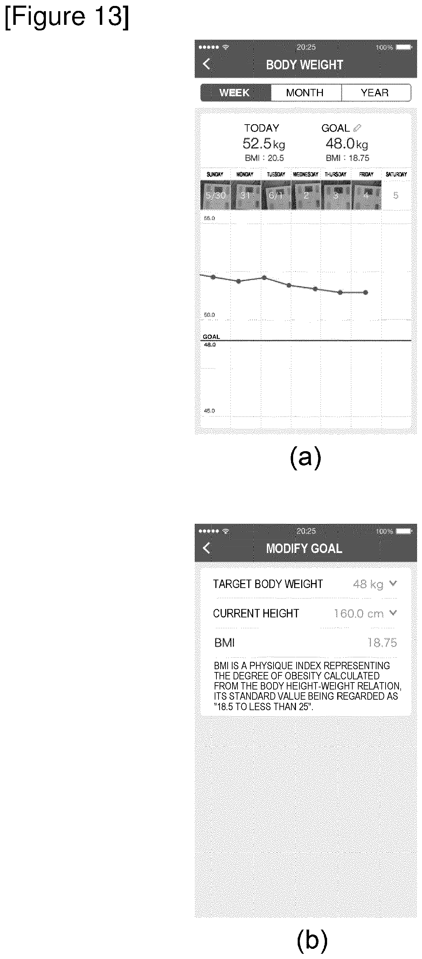

Moreover, for example, when the user selects the "body weight" in the display of the lifestyle habit log shown in Figure 10, the transition of the body weight for seven days among which "June 5 (Saturday)" is the final day can also be visualized and displayed, and moreover, the target value thereof can also be displayed, as shown in Figure 13(a). Furthermore, the transition of the body weight can also be displayed for the period of a "month" or a "year" in addition to a "week". Here, as shown in Figure 13(b), the user can modify the body weight as the goal, and also, the height and the BMI.

As above, the lifestyle habit log can be displayed for every period, and moreover, the target value can be flexibly modified.

Note that the user can share the lifestyle habit information as the lifestyle habit log, associating it with the date and/or the period, with other users via the chat communication interface, and the lifestyle habit logs of the users can be compared and displayed. Thereby, the predetermined missions for achieving the goal can be continued with reference to the lifestyle habit logs among the users.

Furthermore, in the server terminal 100 or another terminal, the lifestyle habit log(s) of one or a plurality of users can be analyzed to provide advice and to provide guidance for achieving the goal. For example, via the chat communication interface of the team, a chat bot, as an instructor, can encourage a user slowing down its actions and/or can advise a user falling behind with comparison of the lifestyle habit logs of a plurality of users.

Note that while in the aforementioned embodiment, the number of steps, a body weight, meals, consumed calories, and a sleeping time are exemplarily represented as the lifestyle habit information, the lifestyle habit information can also include, with no limitation, health information required for diabetes treatment, biological information (such, for example, as a blood glucose level, an arterial pressure, a heartbeat, and blood ketone bodies), and/or information regarding study for university entrance examination and learning of English, for example, as long as they are information required for things that need continuous habits.

While some embodiments according to the invention have been described above, they can be implemented in various other modes, and they can be implemented with various omissions, substitutions, and alterations. These embodiments, their modifications, and omitted, substituted and altered ones are included in the technical scope of the claims and the scope of its equivalents.

### Reference Signs List

- 1: System
- 100: Server terminal
- 110: Communication unit
- 120: Storage unit
- 130: Control Unit
- 200: User terminal
- NW: Network

## Claims

1. A providing method of lifestyle habit improvement for a user belonging to a team in which a plurality of users communicate via an interface for chat communication, the method comprising, by a server terminal:
accepting, from a user terminal, a request for selecting a desired team by a user;
enrolling the user in the team.

2. The providing method according to Claim 1, comprising, by the server terminal:
accepting, from the user terminal, a request for displaying a lifestyle habit log of the user;
generating the lifestyle habit log on the basis of the lifestyle habit information stored in a user data storage; and
displaying the lifestyle habit log.

3. The providing method according to Claim 1, wherein
the lifestyle habit information at least includes any item of information of the number of steps, a body weight, a meal, consumed calories and sleep of the user.

4. The providing method according to Claim 1, comprising
generating a lifestyle habit log for every predetermined period.

5. The providing method according to Claim 1, wherein
in the lifestyle habit method, the team is
generated by an individual user or a corporate user, and is
at least associated with one category of a plurality of categories, and
the category is able to be generated by the corporate user.
